# EUROPÄISCHE PATENTANMELDUNG

(11) **EP 2 762 490 A1**
(43) Veröffentlichungstag der Anmeldung: **06.08.2014**
(21) Anmeldenummer: 13153472.9
(22) Anmeldetag: 31.01.2013
(51) Int. Cl.: C07K 16/04, A23C 9/142, A23L 3/34, A23J 3/08

(54) **Verfahren zur Gewinnung von Immunoglobulinen aus Kolostralmilch**

(71) Anmelder: DMK Deutsches Milchkontor GmbH, 27404 Zeven (DE)
(72) Erfinder: Döring, Sven-Rainer, 27404 Zeven (DE)
(74) Vertreter: Fabry, Bernd

(57) **Zusammenfassung**

Vorgeschlagen wird ein Verfahren zur Gewinnung von Immunoglobulinen, bei dem man
(a) Kolostralmilch der Tage 0 bis 7 einer Temperaturbehandlung unterwirft und den Rahm abtrennt,
(b) die so erhaltene Magermilch einer sterilen Mikrofiltration unterwirft und dabei ein erstes Retentat R1, das das Casein enthält, und ein erstes Permeat P1 herstellt,
(c) das erste Permeat P1 einer Ultrafiltration unterwirft und dabei ein zweites Permeat P2, das Lactose und Mineralien enthält, und ein zweites Retentat R2 herstellt, in welchem die Immunoglobuline angereichert sind.

## Beschreibung

### Gebiet der Erfindung

Die Erfindung befindet sich auf dem Gebiet der pharmazeutischen Zubereitungen und betrifft ein Verfahren zur Gewinnung von Immunoglobulinen, bei dem man Kolostralmilch einer gekoppelten Mikro- und Ultrafiltration unterwirft.

### Stand der Technik

Das Kolostrum ist die Erstmilch für Säugetiere, die von der weiblichen Milchdrüse produziert wird, um das Neugeborene in den ersten Tagen optimal zu ernähren. Es wird auch als Vormilch, Biestmilch, Erstmilch und insbesondere Kolostralmilch bezeichnet und besteht aus Proteinen, Enzymen, Vitaminen, Mineralien, Wachstumsfaktoren, Aminosäuren und Antikörpern. Auf diese Weise wird die Stärkung und die Immunabwehr des Jungtiers unterstützt. Kolostralmilch gilt als gesundes Lebensmittel.

Es gibt zahlreiche Studien zur Wirksamkeit von Kolostrum. Unter anderem soll Kolostrum einen gewissen Schutz vor Infektionskrankheiten bieten (vgl. Cesarone, et al., Clinical and Applied Thrombosis/Hemostasis 13 (2), S. 130-136 (2007**)** und die Wundheilung sowie die Regeneration geschädigter Darmschleimhaut unterstützen. Kolostrum wird auch zur Mitbehandlung von Allergien, Bluthochdruck, Diabetes mellitus und Depressionen eingesetzt. Belege für die Wirksamkeit der Anwendung bei diesen Indikationen fehlen bisher.

Kolostralmilch von Kühen ist als Rohstoffquelle speziell für Immunoglobuline ausgesprochen interessant. Allerdings ist es bisher nur technisch sinnvoll, die innerhalb der ersten 24 bis längstens 48 Stunden nach der Geburt produzierte Milch für eine Abtrennung zu nutzen, da anschließend der Globulinghalt so stark abfällt, dass eine Anreicherung ökonomisch nicht mehr sinnvoll ist.

In der EP 0410272 A1 (Biotest) wird die Herstellung eines Immunoglobulinkonzentrats beschrieben, bei dem man Kolostralmilch aus den ersten 30 Stunden nach der Geburt entrahmt, pasteurisiert, sterilisiert einer Ultrafiltration unterwirft und dann sprühtrocknet. Das Verfahren arbeitet jedoch nur dann wirtschaftlich, wenn man Kolostralmilch mit bereits vergleichbar hohem Immunglobulingehalt einsetzt. Für eine Milch, die erst nach 48 Stunden gewonnen wird, ist das Anreicherungsverfahren ungeeignet.

Gegensand der EP 0918464 B1 (Achenbach) ist ein Verfahren zur Herstellung eines entcaseinierten Kolostralmilchproduktes, wobei Rohkolostrum entfettet und derart angesäuert wird, dass Casein in Lösung bleibt und eine Ultrafiltration mit einer Ausschlussmolekularmasse von mindestens 10⁶ Dalton durchgeführt wird. Das so erhaltene Kolostralmilchprodukt eignet sich besonders als Zusatz für Arzneimittel, Nahrungsergänzungsmittel Getränke, Kindernahrung, Tiernahrung, als Getränke im Intensivsportbereich um Muskelzellschutz beziehungsweise zur Abkürzung der muskulären Erholungsphase sowie zur Prophylaxe und der Therapie von bakteriellen, viralen und mykotischen Infektionen. Mit der Anreicherung von Immunoglobulinen hat diese Veröffentlichung indes nichts zu tun.

In der EP 0410272 A1 (Biotest) wird die Herstellung eines Immunoglobulinkonzentrats beschrieben, bei dem man Kolostralmilch aus den ersten 30 Stunden nach der Geburt entrahmt, pasteurisiert, sterilisiert einer Ultrafiltration unterwirft und dann sprühtrocknet.

Des Weiteren ist aus der AT 511308 A2 (Vitalplus) ein Verfahren zur Herstellung eines lactosearmen oder lactosefreien Kolostralmilchprodukts bekannt, wobei Rohkolostrum entfettet und entkaseiniert wird und das nach einer Ultrafiltration, erhaltene hochmolekulare Inhaltsstoffe enthaltende Produkt einer Sterilisation und/oder Sterilfiltration unterworfen wird. Das Permeat der Ultrafiltration, enthaltend insbesondere niedermolekulare Inhaltsstoffe des Rohcolostrums und Lactose, wird einer Lactosespaltung unterworfen und die niedermolekularen Inhaltsstoffe und die Lactosespaltungsprodukte anschließend dem die hochmolekularen Inhaltsstoffe enthaltenden Produkt vor bzw. bei der Sterilisation bzw. Sterilfiltration wieder beigemengt. Das Verfahren zielt jedoch nicht auf die Anreicherung und Gewinnung von Immunoglobulinen ab.

Schließlich ist aus der EP 0085005 B1 (Fromageries Bel) ein Verfahren zur Abtrennung von Immunoglobuline aus Kolostralmilch bekannt, bei dem man die Kolostralmilch einer Fraktionierung mittels Elektrophorese unterwirft, die mit den Immunoglobulinen angereicherte Fraktion einer Elektrophorese unterwirft, und die dabei erhaltene erste angereicherte Fraktion über einen Anionenaustauscher gibt, um auf diese Weise eine zweite, weiter aufkonzentrierte Fraktion mit Immunoglobulinen zu erhalten.

Die Aufgabe der vorliegenden Erfindung hat somit darin bestanden, ein Verfahren zur Verfügung zu stellen, mit dessen Hilfe man den Gehalt an Imunoglobulinen auch in Kolostralmilch, die während der Tage 2 bis 7 nach der Niederkunft gewonnen wird, mit einem möglichst geringen technischen Aufwand anreichern kann.

### Beschreibung der Erfindung

Gegenstand der Erfindung ist ein Verfahren zur Gewinnung von Immunoglobulinen, bei dem man
(a) Kolostralmilch der Tage 0 bis 7 einer Temperaturbehandlung unterwirft und den Rahm abtrennt,
(b) die so erhaltene Magermilch einer sterilen Mikrofiltration unterwirft und dabei ein erstes Retentat R1, das das Casein enthält, und ein erstes Permeat P1 herstellt,
(c) das erste Permeat P1 einer Ultrafiltration unterwirft und dabei ein zweites Permeat P2, das Lactose und Mineralien enthält, und ein zweites Retentat R2 herstellt, in welchem die Immunoglobuline, speziell die wertvollen Spezies IgG₁, IgA und IgM angereichert sind.

Überraschenderweise wurde gefunden, dass auf diese Weise auch unter Verwendung von Kolostralmilch, die bis zu 7 Tagen nach der Niederkunft gewonnen worden ist, eine Fraktion erhalten wird, die bis zu 15 Gew.-% Immunoglobuline enthält. Diese Fraktion kann dann vorzugsweise mit Hilfe von Ionenaustauscher bis auf einen Immunoglobulingehalt von 85 bis 90 Gew.-% angereichert werden. Anschließend können die Produkte durch Trocknung, Dialyse oder Ultrafiltration weiter aufgereinigt und konzentriert werden.

### Thermische Behandlung

Die Wärmebehandlung der Kolostralmilch ("Entkeimung") erfolgt vorzugsweise in Wärmeaustauschern, wobei sich speziell Plattenwärmeaustauscher als besonders geeignet erwiesen haben. An den Wärmeaustauschern liegt ein Temperaturgradient an, der jedoch so gewählt ist, dass die Kolostralmilch für eine Verweilzeit von mindestens 10 und höchstens 30 Sekunden, vorzugsweise etwa 15 Sekunden auf eine Temperatur von etwa 70 bis 80 °C und insbesondere etwa 72 bis 74 °C erhitzt wird.

Die Abtrennung von festen Nichtmilchbestandteilen, wie z.B. somatischen Zellen, Strohresten und dergleichen sowie die Entrahmung des Fettanteils von etwa 4 Gew.-% erfolgt üblicherweise in einem nachgeschalteten Bauteil, vorzugsweise einem Separator. Derartige Bauteile sind aus dem Stand der Technik hinreichend bekannt. In der Milchindustrie sehr verbreitet sind Separatoren der Firma GEA Westfalia Separator GmbH, mit denen die beiden Schritte einzeln oder gemeinsam durchgeführt werden können (http://www.westfalia-separator.com/de/anwendungen/molkereitechnik/milch-molke.html). Entsprechende Bauteile sind beispielsweise auch in der DE 10036085 C1 (Westfalia) beschrieben und dem Fachmann bestens bekannt, so dass es zur Durchführung dieser Verfahrensschritte keiner Erläuterungen bedarf, da sie den allgemeinen Fachwissen zuzurechnen sind.

### Mikrofiltration und Ultrafiltration

Der Kern des erfindungsgemäßen Verfahrens stellt die Kombination von Mikro- und Ultrafiltration dar.

Die Mikrofiltration ist ein Verfahren zur Stoffabtrennung. Der wesentliche Unterschied zwischen der Mikro- und der Ultrafiltration liegt in den verschiedenen Porengrößen und in der unterschiedlichen Membranstruktur wie den Werkstoffen und den beteiligten Filtermaterialien. Eine Filtration durch Membranen mit einer Porengröße < 0,1 µm wird in der Regel Ultrafiltration genannt, während die Filtration bei Porengrößen > 0,1 µm gewöhnlich als Mikrofiltration bezeichnet wird. In beiden Fällen handelt es sich um rein physikalische, d.h. mechanische Membrantrennverfahren, die nach Prinzip des mechanischen Größenausschlusses arbeiten: alle Partikel in den Fluiden, die größer als die Membranporen sind, werden von der Membran zurückgehalten. Treibende Kraft in beiden Trennverfahren ist der Differenzdruck zwischen Zulauf und Ablauf der Filterfläche, der zwischen 0,1 und 10 bar liegt. Der Werkstoff der Filterfläche kann je Einsatzgebiet aus Edelstahl, Kunststoff, Keramik oder textilen Gewebe bestehen. Es gibt verschiedene Erscheinungsformen der Filterelemente: Kerzenfilter, Flachmembranen, Spiralwickelmembranen, Taschenfilter und Hohlfasermodule, die im Sinne der vorliegenden Erfindung alle grundsätzlich geeignet sind.

Nach der Temperaturbehandlung und Entrahmung wird die Magermilch einer Mikrofiltration unterworfen, wobei mit dem so erhaltenen ersten Retentat R1 nicht nur Mikroorganismen, sondern vor allem auch das Casein abgetrennt wird. Die Mikrofiltration erfolgt vorzugsweise mit Hilfe von Membranen, die eine Trenngrenze von 0,2, vorzugsweise von 0,1 µm aufweisen. Dies erweist sich auch vor dem Hintergrund der Entkeimung überraschenderweise als ausreichend, weil zuvor die Mehrzahl der thermolabilen Keime durch die thermische Behandlung abgetrennt und mit dem Separatorschlamm ausgeschleust worden sind. In Kombination dieses Porendurchmessers mit einer Mikrofiltrationseinrichtung, die im Wesentlichen aus einer Spiralwickelmembran, vorzugsweise aber einer Keramikmembran besteht, wird gleichzeitig das Problem der häufigen Verstopfung gelöst. Die Mikrofiltration kann dabei "kalt" oder "warm" durchgeführt werden, d.h. bei Temperaturen im Bereich von 5 bis 70 °C. Vorzugsweise erfolgt die Mikrofiltration jedoch bei Temperaturen im Bereich von 50 bis 55 °C, weil unter diesen Bedingungen eine Denaturierung verhindert und ein maximaler Flux und optimale Permeabilität der Molkenproteine in das Permeat gewährleistet wird.

Das bei der Mikrofiltration erhaltene erste Permeat P1 wird einer Ultrafiltration unterworfen. Dabei fällt ein zweites Permeat P2 an, welches im Wesentlichen Lactose und Mineralien ("Asche") enthält und getrennt aufgearbeitet werden kann. Besonders bewährt haben sich hier Spiralwickelmembranen auf Polymerbasis; andere Materialien können zwar grundsätzlich auch eingesetzt werden, sind jedoch in der Regel wesentlich teurer ohne eine nennenswert höhere Leistung zu zeigen. Die Ultrafiltration erfolgt vorzugsweise mit Membranen die eine Trenngrenze von 0,01 bis 0,1 µm aufweisen. Auch die Ultrafiltration kann "kalt" oder "warm" durchgeführt werden, d.h. bei Temperaturen im Bereich von 5 bis 70 C. Im Fall der Ultrafiltration ist es sinnvoll, die Filtration kalt, d.h. bei Temperaturen im Bereich von 10 bis 15 °C durchzuführen, um eine Schädigung der Molkenproteine zu vermeiden.

### Abtrennung der Immunoglobuline

Die Abtrennung der Immunoglobuline aus dem zweiten Retentat kann nach den Verfahren des Stands der Technik erfolgen, insbesondere nach einem Verfahren, wie es in der eingangs bereits zitierten EP 0085005 B1 beschrieben wird. Vorzugsweise wird das zweite Retentat durch eine Kolonne geleitet, die mit den Anionenaustauscherpartikeln gefüllt ist. Für diesen Zweck kommen beispielsweise poröse Produkte in Frage, die unter der Bezeichnung Spherosil von Rhone-Poulenc vertrieben werden, insbesondere die Typen QMA (stark basischer Austauscher), C (sehr schwach saurer Austauscher), S (stark saurer Austauscher), und X OB 015 (sehr schwach saurer Austauscher), die üblicherweise auf einem Träger aus porösem Silica erhältlich sind. Ebenfalls geeignet ist z.B. auch das Produkt Trisacryl der Firma I.B.F. Die genannten Ionenaustauscher weisen den Vorteil auf, dass sie den bevorzugten pH-Wert, bei dem die Abtrennung erfolgen soll und der zwischen 7 und 8 liegt, nicht verändern. Auf diese Weise wird insbesondere eine Denaturierung der Globuline verhindert. Die Trennung kann bei Temperaturen im Bereich von 2 bis 25 und vorzugsweise 3 bis 10 °C erfolgen. Die Immunoglobuline werden an den Harzen adsorbiert, das austretende Medium ist praktisch frei von Immunoglobulinen. Durch Spülen der Kolonne mit 1N HCl können die Immunoglobuline wieder in Lösung gebracht und praktisch vollständig zurückgewonnen werden. Anschließend kann die so gewonnene Fraktion durch Trocknung, Dialyse, Ultrafiltration und dergleichen bis zu einer Konzentration von 98 bis 100 Gew.-% angereichert werden.

### Beispiele

### Beispiel 1

Kolostralmilch der Tage 0 bis 7 mit einem Immunoglobulingehalt von 3 Gew.-% wurde in einem Plattenwärmeaustauscher über 15 s auf 72 °C erhitzt. Mit Hilfe des angeschlossenen Separators wurde die so pasteurisierte Kolostralmilch vom Rahm und Feststoffen befreit. Die erhaltene Magermilch wurde bei 50 °C einer Mikrofiltration über eine Keramikmembran mit einem Porendurchmesser von 0,1 µm unterworfen und das caseinhaltige Retentat R1 abgetrennt. Das Permeat P1 wurde einer Ultrafiltration bei 10 °C ebenfalls unter Verwendung einer Spiralwickelmembran unterworfen, die einen Porendurchmesser von 0,04 µm aufwies. Auf diese Weise wurde ein zweites Permeat P2 gewonnen, welches Lactose und Mineralien enthielt und getrennt aufgearbeitet wurde, sowie ein zweites Retentat R2, welches einen Immunoglobulingehalt von 15 Gew.-% aufwies.

2 I des Retentats wurden auf eine Anionenaustauscherkolonne gegeben, die mit einem Harz vom Typ Spherosil QMA gefüllt war. Die Temperatur betrug 4 °C, der pH-Wert lag bei 6,5, die Durchflussmenge 300 ml/h. Das durchströmende Medium wies einen Restgehalt an Immunoglobulinen von weniger als 0,5 Gew.-% auf. Anschließend wurde die Kolonne mit 500 ml 1N HCl gewaschen. Die so erhaltene Fraktion wies einen Proteingehalt von 30 Gew.-% sowie bezogen auf die Proteinmenge einen Gehalt von 89 Gew.-% Immunoglobulinen und 11 Gew.-% Albuminen auf. Die nachfolgende Sprühtrocknung führte zu einem trockenen Pulver.

### Vergleichsbeispiel V1

Kolostralmilch der Tage 0 bis 7 mit eine Immunoglobulingehalt von 3 Gew.-% wurde in einem Plattenwärmeaustauscher über 15 s auf 72 °C erhitzt. Mit Hilfe des angeschlossenen Separators wurde die so pasteurisierte Kolostralmilch vom Rahm und Feststoffen befreit. Die erhaltene Magermilch wurde angesäuert, so dass das Casein in Lösung blieb und dann einer Ultrafiltration bei 25 °C unter Verwendung einer Spiralwickelmembran unterworfen, die einen Porendurchmesser von 0,04 µm aufwies. Auf diese Weise wurde ein Permeat P1 gewonnen, welches Lactose und Mineralien enthielt und getrennt aufgearbeitet wurde, sowie ein R1, welches einen Immunoglobulingehalt von 8 Gew.-% aufwies.

2 I des Retentats wurden auf eine Anionenaustauscherkolonne gegeben, die mit einem Harz vom Typ Spherosil QMA gefüllt war. Die Temperatur betrug 4 °C, der pH-Wert lag bei 6,5, die Durchflussmenge 300 ml/h. Das durchströmende Medium wies einen Restgehalt an Immunoglobulinen von weniger als 0,5 Gew.-% auf. Anschließend wurde die Kolonne mit 500 ml 1N HCl gewaschen. Die so erhaltene Fraktion wies einen Proteingehalt von 22 Gew.-% sowie bezogen auf die Proteinmenge einen Gehalt von 71 Gew.-% Immunoglobulinen und 29 Gew.-% Albuminen auf. Die nachfolgende Sprühtrocknung führte zu einem trockenen Pulver.

## Patentansprüche

1. Verfahren zur Gewinnung von Immunoglobulinen, bei dem man
(a) Kolostralmilch der Tage 0 bis 7 einer Temperaturbehandlung unterwirft und den Rahm abtrennt,
(b) die so erhaltene Magermilch einer sterilen Mikrofiltration unterwirft und dabei ein erstes Retentat R1, das das Casein enthält, und ein erstes Permeat P1 herstellt,
(c) das erste Permeat P1 einer Ultrafiltration unterwirft und dabei ein zweites Permeat P2, das Lactose und Mineralien enthält, und ein zweites Retentat R2 herstellt, in welchem die Immunoglobuline angereichert sind.

2. Verfahren nach Anspruch 1, **dadurch gekennzeichnet, dass** man die Kolostralmilch bei 70 bis 80 °C behandelt.

3. Verfahren nach den Ansprüchen 1 und/oder 2, **dadurch gekennzeichnet, dass** man die Kolostralmilch über einen Zeitraum von 10 bis 30 s behandelt.

4. Verfahren nach mindestens einem der Ansprüche 1 bis 3, **dadurch gekennzeichnet, dass** man die Mikrofiltration mit Membranen durchführt, die eine Trenngrenze von 0,1 bis 0,2 µm aufweisen.

5. Verfahren nach mindestens einem der Ansprüche 1 bis 4, **dadurch gekennzeichnet, dass** man die Mikrofiltration mittels einer Keramikmembran durchführt.

6. Verfahren nach mindestens einem der Ansprüche 1 bis 5, **dadurch gekennzeichnet, dass** man die Mikrofiltration bei Temperaturen im Bereich von 5 bis 70 °C durchführt.

7. Verfahren nach Anspruch 6, **dadurch gekennzeichnet, dass** man die Mikrofiltration bei Temperaturen in Bereich von 50 bis 55 °C durchführt.

8. Verfahren nach mindestens einem der Ansprüche 1 bis 7, **dadurch gekennzeichnet, dass** man die Ultrafiltration mit Membranen durchführt, die eine Trenngrenze von 0,01 bis 0,1 µm aufweisen.

9. Verfahren nach mindestens einem der Ansprüche 1 bis 8, **dadurch gekennzeichnet, dass** man die Ultrafiltration mittels einer Spiralwickelmembran durchführt.

10. Verfahren nach mindestens einem der Ansprüche 1 bis 9, **dadurch gekennzeichnet, dass** man die Ultrafiltration bei Temperaturen im Bereich von 5 bis 70 °C durchführt.

11. Verfahren nach Anspruch 10, **dadurch gekennzeichnet, dass** man die Ultrafiltration bei Temperaturen in Bereich von 10 bis 15 °C durchführt.

12. Verfahren nach mindestens einem der Ansprüche 1 bis 11, **dadurch gekennzeichnet, dass** man die Immunoglobuline aus dem zweiten Retentat durch Behandlung mit einem Anionenaustauscher abtrennt.

13. Verfahren nach Anspruch 12, **dadurch gekennzeichnet, dass** man die Abtrennung bei einer Temperatur im Bereich von 2 bis 25 °C durchführt.

14. Verfahren nach den Ansprüchen 12 und/oder 13, **dadurch gekennzeichnet, dass** man die Abtrennung bei einem pH-Wert von 6 bis 7 durchführt.
